# EUROPÄISCHE PATENTSCHRIFT

(11) **EP 1 233 067 B1**
(45) Veröffentlichungstag und Bekanntmachung des Hinweises auf die Patenterteilung: **26.05.2004**
(21) Anmeldenummer: 02002036.8
(22) Anmeldetag: 07.02.2002
(51) Int. Cl.: C12P 13/00

(54) **Verfahren zur fermentativen Herstellung von O-Acetyl-L-Serin**
Method for the fermentative production of O-acetyl-L-serine
Méthode pour la production fermentaire de O-acetyl-L-serine

(30) Priorität: 15.02.2001 DE 10107002
(43) Veröffentlichungstag der Anmeldung: 21.08.2002
(73) Patentinhaber: Consortium für elektrochemische Industrie GmbH, 81379 München (DE)
(72) Erfinder: Maier, Thomas, Dr., 85221 Dachau (DE); Dassler, Tobias, 81825 München (DE); Böck, August, Prof. Dr., 82260 Geltendorf (DE)
(74) Vertreter: Potten, Holger

(56) Entgegenhaltungen:
- EP-A- 0 885 962
- WO-A-97/15673
- DASSLER T ET AL: "IDENTIFICATION OF A MAJOR FACILITATOR PROTEIN FROM ESCHERICHIA COLI INVOLVED IN EFFLUX OF METABOLITES OF THE CYSTEINE PATHWAY" MOLECULAR MICROBIOLOGY, BLACKWELL SCIENTIFIC, OXFORD, GB, Bd. 36, Nr. 5, Juni 2000 (2000-06), Seiten 1101-1112, XP000992212 ISSN: 0950-382X
- TAI ET AL: "Acid-Base Chemical Mechanism of O-Acetylserine Sulfhydrylases-A and -B from pH Studies" BIOCHEMISTRY, Bd. 34, 1995, Seiten 12311-12322, XP001053699 USA
- KREDICH N M ET AL: "THE ENZYMIC SYNTHESIS OF L-CYSTEINE IN ESCHERICHIA COLI AND SALMONELLA TYPHIMURIUM" JOURNAL OF BIOLOGICAL CHEMISTRY, THE AMERICAN SOCIETY OF BIOLOGICAL CHEMISTS, INC.,, US, Bd. 241, Nr. 21, 10. November 1966 (1966-11-10), Seiten 4955-4965, XP000618470 ISSN: 0021-9258
- NEIDHARDT F.C ET AL: 'Escherichia coli and Salmonella, cellular and molecular biology. Volume 2, p1572', 1996, ASM PRESS, US, WASHINGTON

## Beschreibung

Die Erfindung betrifft Verfahren zur Herstellung von O-Acetyl-L-Serin mittels Fermentation.

Fermentative Prozesse zur Herstellung von Aminosäuren sind heute weit verbreitet. Dabei handelt es sich insbesondere um Herstellverfahren für die wirtschaftlich stark relevanten Vertreter der zwanzig proteinogenen Aminosäuren wie z. B. L-Glutaminsäure, L-Lysin, L-Threonin. Es mehren sich aber auch Berichte über Prozesse für die Herstellung von proteinogenen Aminosäuren mit kleineren Märkten im Bereich von 1000 bis 10000 Jahrestonnen wie z. B. L-Phenylalanin, L-Cystein.

Entsprechende Verfahren zur Herstellung von biosynthetischen Vorläufern der zwanzig proteinenogenen Aminosäuren sind dagegen kaum bekannt. Gerade diese Vorläufer können aber interessante Produkte darstellen, da sie häufig chirale Zentren besitzen und als Bausteine für die Synthese von Pharmawirkstoffen dienen können. In der Patentanmeldung WO 00/44923 ist zum Beispiel die Herstellung von Shikimisäure, einem Intermediat der Biosynthese von aromatischen Aminosäuren, beschrieben. Ein weiteres Beispiel ist die Anmeldung EP 0994190 A2, in der die fermentative Produktion von L-Homoserin, einem Vorläufer von L-Methionin, berichtet wird.

O-Acetyl-L-Serin ist ein biosynthetischer Vorläufer von L-Cystein. Es stellt seinerseits eine Aminosäure dar und entsteht im Stoffwechsel von Bakterien und Pflanzen durch die Acetylierung von L-Serin an der Hydroxylfunktion. Diese Reaktion wird vom Enzym Serin-O-Acetyl-Transferase [EC 2.3.1.30] katalysiert, das vom cysE-Gen kodiert wird. O-Acetyl-Serin wird in der Zelle weiter zu L-Cystein umgesetzt. Dabei wird eine Substitution der Acetat-Funktion gegen eine Thiol-Funktion vorgenommen.

Probleme bei einer fermentativen Herstellung von O-Acetyl-L-Serin ergeben sich aus der Tatsache, dass die Substanz sehr labil ist und bei pH-Werten über 4,0 zum N-Acetyl-L-Serin isomerisiert. Die Geschwindigkeit der Reaktion besitzt bei einem pH-Wert von 7,6 eine Rate von 1 % x min⁻¹ (Tai et al., 1995, Biochemistry 34: 12311-12322). Dies bedeutet, dass unter solchen Bedingungen nach einem Fermentationsprozeß, der üblicherweise mindestens eineinhalb Tage geführt wird, keine signifikanten Mengen O-Acetyl-Serin nachzuweisen sind. Die Isomerisierungsreaktion ist irreversibel und steigt in ihrer Geschwindigkeit mit zunehmendem pH-Wert weiter an. Im Gegensatz zu O-Acetyl-L-Serin kann N-Acetyl-L-Serin aufgrund der Blockierung der Amino-Funktion nicht mehr für Peptidsynthesen verwendet werden.

Ein weiteres Problem ist, dass der O-Acetyl-L-Serin-Spiegel in der Zelle sehr niedrig ist und einer starken Regulation unterliegt. Einerseits ist die Serin-Acetyl-Transferase durch L-Cystein allosterisch gehemmt und damit ist in Anwesenheit von µM-Konzentrationen an L-Cystein keine Synthese von O-Acetyl-L-Serin möglich. Andererseits wirkt das Isomerisierungsprodukt N-Acetyl-L-Serin als Induktor des Schwefelregulons und führt damit zum schnellen Umsatz von O-Acetyl-L-Serin mit Sulfid zum L-Cystein.

Von Daßler et al. (2000, Mol. Microbiol. 36: 1101-1112) wurde beschrieben, dass Zellen, die das Membranprotein YdeD (=Orf299) überproduzieren, L-Cystein, 2-Methyl-thiazolidin-2,4-dicarbonsäure und auch O-Acetyl-L-Serin in das Kulturmedium ausscheiden. Letzteres konnte jedoch nur in sehr geringen Mengen 0,12 g/l und nur in Schüttelkolbenversuchen nachgewiesen werden. In Fermentationsversuchen, die aufgrund besserer Nährstoffversorgung höhere Ausbeuten ermöglichen, wurde dagegen nur N-Acetyl-L-Serin erhalten. Es wurde diskutiert, dass O-Acetyl-L-Serin nur bei pH-Werten von 4-5 ausreichend stabil ist. Dieser pH-Bereich ist jedoch für eine fermentative Herstellung aufgrund des schlechten Wachstums von neutrophilen Bakterien wie beispielsweise Escherichia coli nicht geeignet.

Die fermentative Herstellung von N-Acetyl-L-Serin bei pH 7,0 unter Verwendung von Orf299 wurde ebenfalls in der Patentanmeldung EP 0885962 A1 beschrieben. Hierbei wurde das orf299-Gen (in der Anmeldung bezeichnet mit SEQ.ID.NO:3) mit geeigneten cysE-Allelen kombiniert. Diese kodierten für Serin-Acetyl-Transferasen, die einer verminderten Feedback-Hemmung durch L-Cystein unterliegen. Dadurch wurde eine verstärkte Produktion von O-Acetyl-L-Serin in der Zelle erreicht und letztendlich aufgrund der schnellen Isomerisierung N-Acetyl-L-Serin akkumuliert.

Der alleinige Einsatz von cysE-Allelen mit verminderter Feedback-Hemmumg wie in der Anmeldung WO 97/15673 beschrieben führt ebenfalls nicht zur Akkumulation von O-Acetyl-L-Serin. Zwar wird intrazellulär eine vermehrte Bildung von O-Acetyl-Serin erzielt. Extrazellulär - und damit erst als Produkt greifbar - konnte bei dieser Vorgehensweise aber lediglich L-Cystein nachgewiesen werden.

Ein großes Problem bei einer Produktion von O-Acetyl-L-Serin stellt außerdem die von Daßler et al. (2000, Mol. Microbiol. 36: 1101-1112) beschriebene Tatsache dar, dass die Überproduktion von Orf299 zu einer starken Beeinträchtigung des Bakterienwachstums führt. Dies beruht auf einer fehlenden Induktion des Schwefelregulons aufgrund des intrazellulären Mangels am Induktor N-Acetyl-L-Serin.

Aufgabe der Erfindung war es, ein fermentatives Verfahren bereitzustellen, das trotz der Instabilität von O-Acetyl-L-Serin und der negativen physiologischen Konsequenzen eines effizienten O-Acetyl-L-Serin-Exports aus der Zelle hohe Ausbeuten an O-Acetyl-L-Serin liefert.

Die Aufgabe wurde dadurch gelöst, dass ein von einem Wildtyp abgeleiteter Mikroorganismen-Stamm mit einer im Vergleich zum Wildtyp erhöhten endogenen O-Acetyl-L-Serin-Bildung und einem im Vergleich zum Wildtyp verstärkten O-Acetyl-L-Serin-Efflux in einem Fermentationsmedium kultiviert wird, dadurch gekennzeichnet, dass im Fermentationsmedium ein pH-Wert im Bereich von 5,5 bis 6,0 eingestellt wird.

Es wurde nämlich überraschenderweise gefunden,
- dass ein wie oben gekennzeichneter Mikroorganismenstamm in großen Mengen O-Acetyl-L-Serin exkretiert,
- dass O-Acetyl-L-Serin bei pH-Werten von 5,5 bis 6,0 im Fermentationsmedium ausreichend stabil ist und
- dass zugleich die oben genannten physiologischen Probleme einer O-Acetyl-L-Serin-exkretierenden Zelle durch eine vermehrte Bereitstellung von O-Acetyl-L-Serin mittels feedbackresistenter cysE-Allele weitgehend behoben werden können.

Mikroorganismenstämme, die im erfindungsgemäßen Verfahren eingesetzt werden können, zeichnen sich dadurch aus, dass sie
- eine erhöhte endogene Bildung von O-Acetyl-L-Serin aufweisen und
- über einen verstärkten O-Acetyl-L-Serin-Efflux verfügen.

Solche Stämme sind im Stand der Technik bekannt. Eine erhöhte endogene Bildung von O-Acetyl-L-Serin kann dadurch erreicht werden, dass modifizierte cysE-Allele wie beispielsweise in
- WO 97/15673 oder
- Nakamori S. et al., 1998, Appl. Env. Microbiol. 64: 1607-1611 oder
- Takagi H. et al., 1999, FEBS Lett. 452: 323-327 beschrieben, in einen Mikroorganismenstamm eingebracht werden.

Diese cysE-Allele kodieren für Serin-O-Acetyl-Transferasen, die einer verminderten Feedback-Hemmung durch Cystein unterliegen. Dadurch wird die Bildung von O-Acetyl-L-Serin weitgehend vom Cystein-Spiegel der Zelle entkoppelt.

Ein verstärkter O-Acetyl-L-Serin-Efflux kann durch erhöhte Expression eines Efflux-Gens, dessen Genprodukt den Export von O-Acetyl-L-Serin bewirkt, erzielt werden.

Ein besonders bevorzugtes solches Efflux-Gen stellt das von Daßler et al. (2000, Mol. Microbiol. 36: 1101-1112) und in EP 0885962 A1 beschriebene ydeD-Gen dar.

Die modifizierten cysE-Allele bzw. das Efflux-Gen können im verwendeten Stamm in Einzelkopie oder auch in erhöhter Kopienzahl vorliegen. Sie können chromosomal kodiert sein oder auf selbstreplizierenden Elementen wie z. B. Plasmiden lokalisiert sein.

Eine verstärkte Expression der Gene kann beispielsweise durch Verwendung geeigneter Promotorsysteme, die dem Fachmann bekannt sind, erfolgen.

In einer bevorzugten Ausführungsform der vorliegenden Erfindung wird ein Mikroorganismus enthaltend ein cysE-Allel bzw. ein ydeD-Gen mit nativem bzw. dem gapDH-Promotor auf einem Plasmid mit mittlerer Kopienzahl eingesetzt. Ein solches Konstrukt ist beispielsweise pACYC184-cysEX-GAPDH-ORF306, das in EP 0885962 A1 eingehend beschrieben ist.

Zur Herstellung solcher Stämme sind prinzipiell alle Mikroorganismenstämme geeignet, die genetischen Methoden zugänglich sind und in einem Fermentationsprozess gut kultivierbar sind. Bevorzugt werden Bakterien der Familie der Enterobacteriaceen verwendet. Besonders bevorzugt werden Organismen der Art Escherichia coli verwendet. Die Herstellung solcher Stämme ist in den genannten Schriften beschrieben und nicht Teil der vorliegenden Erfindung.

Für das erfindungsgemäße Verfahren geeignete Stämme eignen sich - wie in EP 0885962 A1 beschrieben - auch zur Herstellung von Cystein und Cystein-Derivaten. Für letzters ist jedoch eine ausreichende Zuführung einer anorganischen Schwefel-Quelle wie z. B. Sulfat oder Thiosulfat notwendig, um optimale Mengen an L-Cystein oder eines Derivates davon zu erzielen.

Im erfindungsgemäßen Verfahren wird jedoch keine Schwefelquelle während der Fermentation zudosiert, da die weitere Umsetzung von O-Acetyl-L-Serin zu Cystein unerwünscht ist. Es muß lediglich sichergestellt werden, dass im Medium eine ausreichende Menge einer Schwefelquelle (z. B. Sulfat, Thiosulfat) vorhanden ist, damit der Bedarf an Cystein für die Proteinsynthese der Zelle gedeckt wird. Die ausreichende Menge im Nährmedium beträgt vorzugsweise 5 bis 50 mM Schwefel.

Das erfindungsgemäße Verfahren zur Herstellung von O-Acetyl-L-Serin mit Hilfe eines Mikroorganismenstammes wird in einem Fermenter in an und für sich bekannter Art und Weise aber unter Einstellen von unüblich niedrigen pH-Werten während der Fermentation durchgeführt.

Die Anzucht des Mikroorganismenstammes im Fermenter erfolgt als kontinuierliche Kultur, als batch-Kultur oder vorzugsweise als fed-batch-Kultur. Besonders bevorzugt wird eine C-Quelle während der Fermentation kontinuierlich zudosiert.

Als C-Quelle dienen vorzugsweise Zucker, Zuckeralkohole oder organische Säuren. Besonders bevorzugt werden im erfindungsgemäßen Verfahren als C-Quellen Glukose, Laktose oder Glycerin eingesetzt.

Bevorzugt ist die Dosierung der C-Quelle in einer Form, die gewährleistet, dass der Gehalt im Fermenter während der Fermentation in einem Bereich von 0,1 - 50 g/l gehalten wird. Besonders bevorzugt ist ein Bereich von 0,5 - 10 g/l.

Als N-Quelle werden im erfindungsgemäßen Verfahren vorzugsweise Ammoniak, Ammoniumsalze oder Proteinhydrolysate verwendet. Bei Verwendung von Ammoniak als Korrekturmittel zur pH-Statisierung wird während der Fermentation regelmäßig diese N-Quelle nachdosiert.

Als weitere Medienzusätze können Salze der Elemente Phosphor, Chlor, Natrium, Magnesium, Stickstoff, Kalium, Calcium, Eisen und in Spuren (d. h. in µM Konzentrationen) Salze der Elemente Molybdän, Bor, Kobalt, Mangan, Zink und Nickel zugesetzt werden.

Des Weiteren können organische Säuren (z. B. Acetat, Citrat), Aminosäuren (z. B. Isoleucin) und Vitamine (z. B. B1, B6) dem Medium zugesetzt werden.

Als komplexe Nährstoffquellen können z. B. Hefeextrakt, Maisquellwasser, Sojamehl oder Malzextrakt zum Einsatz kommen.

Die Inkubationstemperatur beträgt 15 - 45 °C. Bevorzugt ist eine Temperatur zwischen 30 - 37 °C.

Die Fermentation wird vorzugsweise unter aeroben Wachstumsbedingungen durchgeführt. Der Sauerstoffeintrag in den Fermenter erfolgt mit Pressluft oder mit reinem Sauerstoff.

Mikroorganismen, die nach dem beschriebenen Verfahren fermentiert werden, sezernieren in einer Fermentationszeit von 1 bis 3 Tage O-Acetyl-L-Serin mit hoher Effizienz in das Kulturmedium.

Die folgenden Beispiele dienen der weiteren Erläuterung der Erfindung. Der Bakterienstamm *Escherichia coli* W3110 / pA-CYC184-cysEX-GAPDH-ORF306, der für die Ausführung der Beispiele verwendet wurde, wurde bei der DSMZ (Deutsche Sammlung für Mikroorganismen und Zellkulturen GmbH, D-38142 Braunschweig) unter der Nummer DSM 13495 gemäß Budapester Vertrag hinterlegt.

### Beispiel 1: Isomerisierung von O-Acetyl-L-Serin zu N-Acetyl-L-Serin

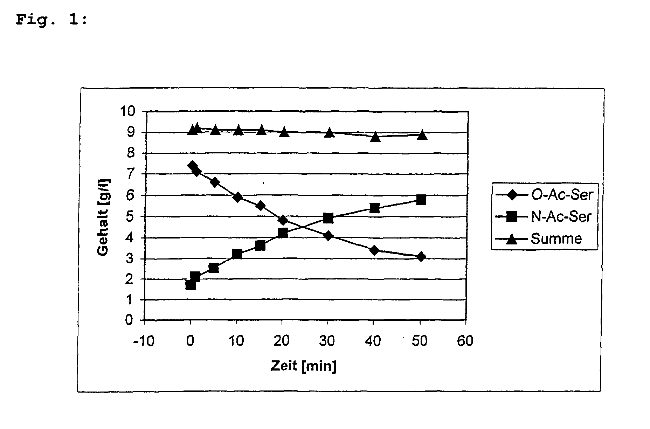

Um einen genaueren Eindruck über die Isomerisierungsreaktion unter fermentationsnahen Bedingungen zu gewinnen wurden 0,9 g O-Acetyl-L-Serin in 100 ml Fermentationsmedium (siehe Beispiel 3) eingesetzt. Der pH-Wert wurde daraufhin mit 25 %igem Ammoniak auf pH 7,0 eingestellt und bei einer Reaktionstemperatur von 32 °C wurden zu verschiedenen Zeitpunkten Proben entnommen. Diese wurden durch reversed phase HPLC an einer LUNA 5 µ C18(2)-Säule (Phenomenex, Aschaffenburg, Deutschland) analysiert. Als Eluent diente verdünnte Phosphorsäure (0,1 ml konz. Phosphorsäure / 1) bei einer Flußrate von 0,5 ml/min. Die Ergebnisse sind in Fig. 1 dargestellt.

### Beispiel 2: Vorkultur des Produktionsstammes

Als Vorkultur für die Fermentation wurden 20 ml LB-Medium (10 g/l Trypton, 5 g/l Hefeextrakt, 10 g/l NaCl), das zusätzlich 15 mg/l Tetracyclin enthielt, mit dem Stamm W3110 / pACYC184-cysEX-GAPDH-ORF306 (beschrieben in EP 0885962 A1) beimpft und bei 30 °C und 150 rpm in einem Schüttler inkubiert. Nach sieben Stunden wurde der gesamte Ansatz in 100 ml SM1-Medium (12 g/l K₂HPO₄; 3 g/l KH₂PO₄; 5 g/l (NH₄)₂SO₄; 0,3 g/l MgSO₄ x 7 H₂O; 0,015 g/l CaCl₂ x 2 H₂O; 0,002 g/l FeSO₄ x 7 H₂O; 1 g/l Na₃Citrat x 2 H₂O; 0,1 g/l NaCl; 1 ml/l Spurenelementlösung bestehend aus 0,15 g/l Na₂MoO₄ x 2 H₂O; 2,5 g/l Na₃BO₃; 0,7 g/l CoCl₂ x 6 H₂O; 0,25 g/l CuSO₄ x 5 H₂O; 1,6 g/l MnCl₂ x 4 H₂O; 0,3 g/l ZnSO₄ x 7 H₂O), das mit 5 g/l Glukose; 0,5 mg/l Vitamin B₁ und 15 mg/l Tetracyclin supplementiert wurde, überführt. Die weitere Inkubation erfolgte bei 30 °C für 17 Stunden bei 150 rpm.

### Beispiel 3: Fermentative Herstellung von O-Acetyl-L-Serin

Als Fermenter diente ein Biostat M-Gerät der Firma Braun Biotech (Melsungen, D) mit einem maximalen Kulturvolumen von 2 1. Mit der in Beispiel 2 beschriebenen Vorkultur (optische Dichte bei 600 nm von ca. 3) wurde der Fermenter mit 900 ml Fermentationsmedium (15 g/l Glukose; 10 g/l Trypton; 5 g/l Hefeextrakt; 5 g/l (NH₄)₂SO₄; 1,5 g/l KH₂PO₄; 0,5 g/l NaCl; 0,3 g/l MgSO₄ x 7 H₂O; 0,015 g/l CaCl₂ x 2 H₂O; 0,075 g/l FeSO₄ x 7 H₂O; 1 g/l Na₃Citrat x 2 H₂O und 1 ml/l Spurenelementlösung s.o., 5 mg/l Vitamin B1 und 15 mg/l Tetracyclin, eingestellt auf pH 6,0 mit 25 % Ammoniak) beimpft. Während der Fermentation wurde eine Temperatur von 32 °C eingestellt und der pH-Wert durch Zudosierung von 25 % Ammoniak bei einem Wert von 6,0 konstant gehalten. Die Kultur wurde mit entkeimter Druckluft bei 1,5 vol/vol/min begast und mit einer Rührerdrehzahl von 200 rpm gerührt. Nach Absinken der Sauerstoffsättigung auf einen Wert von 50 % wurde die Drehzahl über ein Kontrollgerät bis zu einem Wert von 1200 rpm erhöht, um 50 % Sauerstoffsättigung zu erhalten (Bestimmt mit einer pO₂-Sonde, kalibriert auf 100% Sättigung bei 900 rpm). Sobald der Glukose-Gehalt im Fermenter von anfänglich 15 g/l auf ca. 5-10 g/l abgesunken war, erfolgte eine Zudosierung einer 56 % Glukose-Lösung. Die Fütterung erfolgte mit einer Flußrate von 6-12 ml/h, wobei die Glukosekonzentration im Fermenter zwischen 0,5 - 10 g/l konstant gehalten wurde. Die Glukose-Bestimmung wurde mit dem Glukoseanalysator der Firma YSI (Yellow Springs, Ohio, USA) durchgeführt. Die Fermentationsdauer betrug 28 Stunden. Nach dieser Zeit wurden Proben entnommen und die Zellen durch Zentrifugation vom Kulturmedium abgetrennt. Die resultierenden Kulturüberstände wurden durch reversed phase HPLC wie in Beispiel 1 beschrieben analysiert. Die Tabelle 1 zeigt die erzielten Gehalte der Hauptstoffwechselprodukte im Kulturüberstand:

**Tabelle 1:**

| **Metabolit** | **Gehalt [g/l]** |
|---|---|
| O-Acetyl-L-Serin | 9,0 |
| N-Acetyl-L-Serin | 4,2 |
| 2-Methyl-thiazolidin-2,4-di-carbonsäure | 1,9 |

Im Bereich bis pH 5,5 wurden ähnliche Werte erzielt.

## Patentansprüche

1. Verfahren zur fermentativen Herstellung von O-Acetyl-L-Serin, bei dem ein Mikroorganismen-Stamm, der ein cysE-Allel kodierend für eine Serin-O-Acetyl-Transferase, die einer verminderten Feedback-Hemmung durch Cystein unterliegt und einen im Vergleich zu einem Wildtyp verstärkten O-Acetyl-L-Serin-Efflux besitzt, in einem Fermentationsmedium kultiviert wird, **dadurch gekennzeichnet, dass** im Fermentationsmedium ein pH-Wert im Bereich von 5,5 bis 6,0 eingestellt wird.

2. Verfahren gemäß Anspruch 1, **dadurch gekennzeichnet, dass** der im Vergleich zum Wildtyp erhöhte O-Acetyl-L-Serin-Efflux durch ein ydeD-Gen erreicht wird.

3. Verfahren gemäß einem der Ansprüche 1 oder 2, **dadurch gekennzeichnet, dass** es sich bei dem Mikroorganismenstamm um Escherichia coli handelt.

4. Verfahren gemäß einem der Ansprüche 1 bis 3, **dadurch gekennzeichnet, dass** der Mikroorganismenstamm in einem Fermenter als eine kontinuierliche Kultur, als eine batch-Kultur oder vorzugsweise als eine fed-batch-Kultur angezogen wird.

5. Verfahren gemäß einem der Ansprüche 1 bis 4, **dadurch gekennzeichnet, dass** das Nährmedium eine S-Quelle in einer Konzentration von 5 - 50 mM Schwefel enthält.

6. Verfahren gemäß einem der Ansprüche 1 bis 5, **dadurch gekennzeichnet, daß** eine C-Quelle während der fermentativen Herstellung kontinuierlich zudosiert wird.

7. Verfahren gemäß Anspruch 6, **dadurch gekennzeichnet, dass** die C-Quelle ausgewählt ist aus der Gruppe Zucker, Zuckeralkohole oder organische Säuren.

8. Verfahren gemäß Anspruch 6 oder 7, **dadurch gekennzeichnet, dass** die C-Quelle ausgewählt ist aus der Gruppe Glukose, Laktose oder Glycerin.

9. Verfahren gemäß einem der Ansprüche 6 bis 8, **dadurch gekennzeichnet, dass** die C-Quelle derart zudosiert wird, dass sie während der Fermentation in einem Bereich von 0,1 - 50 g/l vorhanden ist.

10. Verfahren gemäß einem der Ansprüche 1 bis 9, **dadurch gekennzeichnet, dass** als eine N-Quelle Ammoniak, Ammoniumsalze oder Proteinhydrolysate zugesetzt werden.

11. Verfahren gemäß einem der Ansprüche 1 bis 10, **dadurch gekennzeichnet, dass** dem Fermentationsmedium Salze der Elemente Phosphor, Chlor, Natrium, Magnesium, Stickstoff, Kalium, Calcium, Eisen und in Spuren (d.h. in µM Konzentrationen) Salze der Elemente Molybdän, Bor, Kobalt, Mangan, Zink und Nickel zugesetzt werden.

12. Verfahren gemäß einem der Ansprüche 1 bis 11, **dadurch gekennzeichnet, dass** organische Säuren (z.B. Acetat, Citrat), Aminosäuren (z.B. Isoleucin) und Vitamine (z.B. B1, B6) dem Fermentationsmedium zugesetzt werden.

13. Verfahren gemäß einem der Ansprüche 1 bis 12, **dadurch gekennzeichnet, dass** Hefeextrakt, Maisquellwasser, Sojamehl oder Malzextrakt dem Fermentationsmedium zugesetzt werden.

14. Verfahren gemäß einem der Ansprüche 1 bis 13, **dadurch gekennzeichnet, dass** eine Inkubationstemperatur zwischen 15 - 45 °C, bevorzugt zwischen 30 - 37 °C, vorliegt.

15. Verfahren gemäß einem der Ansprüche 1 bis 14, **dadurch gekennzeichnet, dass** es unter aeroben Wachstumsbedingungen durchgeführt wird.

16. Verfahren gemäß Anspruch 15, **dadurch gekennzeichnet, dass** die aeroben Wachstumsbedingungen durch einen Sauerstoffeintrag in den Fermenter mittels Pressluft oder mittels reinem Sauerstoff hergestellt werden.

17. Verfahren gemäß einem der Ansprüche 1 bis 16, **dadurch gekennzeichnet, dass** eine Fermentationszeit von 1 bis 3 Tagen gewählt wird.

## Claims

1. Process for the fermentative preparation of O-acetyl-L-serine, in which a microorganism strain, which has a cysE allele encoding a serine-O-acetyl transferase which is subject to less feedback inhibition by cysteine and has an increased efflux of O-acetyl-L-serine as compared with a wild type, is cultured in a fermentation medium, **characterized in that** a pH in the range from 5.5 to 6.0 is set in the fermentation medium.

2. Process according to Claim 1, **characterized in that** the increased efflux of O-acetyl-L-serine, as compared with the wild type, is achieved using a ydeD gene.

3. Process according to one of Claims 1, **characterized in that** the microorganism strain is Escherichia coli.

4. Process according to one of Claims 1 to 3, **characterized in that** the microorganism strain is grown in a fermenter as a continuous culture, as a batch culture or, preferably, as a fed-batch culture.

5. Process according to one of Claims 1 to 4, **characterized in that** the nutrient medium contains an S source at a concentration of 5 - 50 mM sulfur.

6. Process according to one of Claims 1 to 5, **characterized in that** a C source is metered in continuously during the fermentative preparation.

7. Process according to Claim 6, **characterized in that** the C source is selected from the group sugar, sugar alcohols and organic aids.

8. Process according to Claim 6 or 7, **characterized in that** the C source is selected from the group glucose, lactopse and glycerol.

9. Process according to one of Claims 6 to 8, **characterized in that** the C source is metered in such that it is present in a range of 0.1 - 50 g/l during the fermentation.

10. Process according to one of Claims 1 to 9 **characterized in that** ammonia, ammonium salts or protein hydrolysates is/are added as an N source.

11. Process according to one of Claims 1 to 10, **characterized in that** salts of the elements phosphorous, chlorine, sodium, magnesium, nitrogen, potassium, calcium or iron and, in traces (i.e. in µm concentrations), salts of the elements molybdenum, boron, cobalt, manganese, zinc and nickel, are added to the fermentation medium.

12. Process according to one of Claims 1 to 11, **characterized in that** organic acids (e.g. acetate , citrate), amino acids (e.g. isoleucine) and vitamins (e.g. B1 , B6) are added to the fermentation medium.

13. Process according to one of Claims 1 to 12, **characterized in that** yeast extract, corn steep liquor, soybean meal or malt extract is added to the fermentation medium.

14. Process according to one of Claims 1 to 13, **characterized in that** the incubation temperature is between 15 and 45°C, preferably between 30 and 37'C.

15. Process according to one of Claims 1 to 14, **characterized in that** it is carried out under aerobic growth conditions.

16. Process according to Claim 15, **characterized in that** the aerobic growth conditions are brought about by introducing oxygen into the fermenter by means of compressed air or by means of pure oxygen.

17. Process according to one of Claims 1 to 16, **characterized in that** a fermentation time of from 1 to 3 days is selected.

## Revendications

1. Procédé pour la production par fermentation d'Oacétyl-L-sérine, dans lequel on cultive une souche de microorganismes présentant un allèle cysE codant pour une sérine-O-acétyl-transférase, qui est soumise à une rétro-inhibition réduite par la cystéine et qui présente un efflux d'O-acétyl-L-sérine plus important par rapport à une souche de type sauvage, dans un milieu de fermentation, **caractérisé en ce qu'**on règle dans le milieu de fermentation un pH dans la plage de 5,5 à 6,0.

2. Procédé selon la revendication 1, **caractérisé en ce que** l'efflux plus élevé d'O-acétyl-L-sérine par rapport au type sauvage est obtenu par un gène ydeD.

3. Procédé selon la revendication 1 ou 2, **caractérisé en ce qu'**il s'agit pour la souche de microorganismes d'Escherichia coli.

4. Procédé selon l'une quelconque des revendications 1 à 3, **caractérisé en ce que** la souche de microorganismes est cultivée dans un fermenteur sous forme d'une culture continue, d'une culture en batch ou de préférence, d'une culture en fed-batch.

5. Procédé selon l'une quelconque des revendications 1 à 4, **caractérisé en ce que** le milieu de culture contient une source de S en une concentration de 5 à 50 mM de soufre.

6. Procédé selon l'une quelconque des revendications 1 à 5, **caractérisé en ce qu'**une source de C est dosée en continu pendant la production par fermentation.

7. Procédé selon la revendication 6, **caractérisé en ce que** la source de C est choisie dans le groupe constitué par le sucre, les alcools de sucre ou les acides organiques.

8. Procédé selon la revendication 6 ou 7, **caractérisé en ce que** la source de C est choisie dans le groupe constitué par le glucose, le lactose ou le glycérol.

9. Procédé selon l'une quelconque des revendications 6 à 8, **caractérisé en ce que** la source de C est dosée de telle manière qu'elle est présente pendant la fermentation dans une plage de 0,1 - 50 g/l.

10. Procédé selon l'une quelconque des revendications 1 à 9, **caractérisé en ce qu'**on ajoute comme source de N de l'ammoniaque, des sels d'ammonium ou des protéines hydrolysées.

11. Procédé selon l'une quelconque des revendications 1 à 10, **caractérisé en ce qu'**on ajoute au milieu de fermentation des sels des éléments phosphore, chlore, sodium, magnésium, azote, potassium, calcium, fer et, à l'état de traces (c'est-à-dire en des concentrations de l'ordre de la µM) des sels des éléments molybdène, bore, cobalt, manganèse, zinc et nickel.

12. Procédé selon l'une quelconque des revendications 1 à 11, **caractérisé en ce que** des acides organiques (par exemple acétate, citrate), des acides aminés (par exemple l'isoleucine) et des vitamines (par exemple B1, B6) sont ajoutés au milieu de fermentation.

13. Procédé selon l'une quelconque des revendications 1 à 12, **caractérisé en ce qu'**on ajoute de l'extrait de levure, de l'eau de trempe du maïs, de la farine de soja ou de l'extrait de malt au milieu de fermentation.

14. Procédé selon l'une quelconque des revendications 1 à 13, **caractérisé en ce que** la température d'incubation est située entre 15 et 45°C, de préférence entre 30 et 37°C.

15. Procédé selon l'une quelconque des revendications 1 à 14, **caractérisé en ce qu'**il est réalisé dans des conditions de croissance aérobie.

16. Procédé selon la revendication 15, **caractérisé en ce que** les conditions de croissance aérobie sont réalisées par une introduction d'oxygène dans le fermenteur au moyen d'air comprimé ou au moyen d'oxygène pur.

17. Procédé selon l'une quelconque des revendications 1 à 16, **caractérisé en ce qu'**on choisit un temps de fermentation de 1 à 3 jours.
